Europäisches Patentamt

European Patent Office

Office européen des brevets

(19)

(11) **EP 1 251 167 A2**

(12) **EUROPEAN PATENT APPLICATION**

(43) Date of publication:
23.10.2002 Bulletin 2002/43

(51) Int Cl.⁷: **C12N 9/02**, A23L 1/32, A23C 9/12

(21) Application number: 02008365.5

(22) Date of filing: 12.04.2002

(84) Designated Contracting States:
**AT BE CH CY DE DK ES FI FR GB GR IE IT LI LU MC NL PT SE TR**
Designated Extension States:
**AL LT LV MK RO SI**

(30) Priority: 16.04.2001 US 835804
12.02.2002 US 74116

(71) Applicant: **Valcarce, German A.**
**1425 Buenos Aires (AR)**

(72) Inventor: **Valcarce, German A.**
**1425 Buenos Aires (AR)**

(74) Representative: **Perez Bonal, Bernardo**
**Explanada 8**
**28040 Madrid (ES)**

(54) **Delta 7 and delta 22 cholesterol desaturases from ciliata phylum, and uses**

(57)     A cell free extract from a ciliate microorganism, including protozoo *Tetrahymena*, wherein the extract comprises a homogenate a microsomal fraction, a desaturaseenriched fraction or a mixture thereof and, more particularly the invention provides substantially pure enzymes: Δ7 cholesterol desaturases and Δ22 cholesterol desaturase enzymes.

The present invention provides methods for decreasing the cholesterol content of a foodstuff of animal origin, such as a dairy product. The methods comprise the step of treating a foodstuff by incubating the same with a cell free extract in a effective amount to enable changes in the foodstuff: (a)reduce the level of cholesterol; and (b) increase the level of at least one desaturated cholesterol derivative: Δ7- dehydrocholesterol, Δ22 dehydrocholesterol and/or Δ7,22 bisdehydrocholesterol.

The invention also relates to methods for recovering cholesterol desaturases activities from ciliates, including *Tetrahymena*, and their use in a production of Δ7 dehydrosterol, Δ22 dehydrosterol and Δ7,22 bis dehydrosterol from several sterol sources as cholesterol.

EP 1 251 167 A2

**Description**

## OBJECT OF THE INVENTION

This application relates to the isolation of cell free extracts from a microorganism from Ciliates phylum, such as *Tetrahymenidae* family. One aspect of the present invention is the provision of methods for the conversion of cholesterol present in foodstuffs into cholesterol desaturated derivatives, including provitamin D, using the cell free extracts.

The present invention also relates to a novel sterol desaturases from Ciliata phylum microorganism as for example *Tetrahymena*, and also relates to a process for production of these enzymes. More particularly, the invention relates to methods for recovering of Δ7 cholesterol desaturases and Δ22 cholesterol desaturases from cell free extract.

The invention also relates methods for treating sterol substrates for the production of Δ7 dehydrosterol, Δ22 dehydrosterols and Δ7,22 bis dehydrosterol, in particular Vitamin D precursors and analogs.

## BACKGROUND OF THE INVENTION

Animal milk is a complex mixture of different compounds, including lipids, proteins, minerals, sugars and vitamins. The calcium, phosphate and vitamin D content of milk make it an adequate source of nutrients for bone formation. Milk is also relatively economical, compared to other animal protein sources, and thus it makes a valuable contribution to the human diet (Russof, L.L. (1970) J. Dairy Science 53:1296-1302).

The lipid fraction of milk includes cholesterol, however, which has been implicated as a causative agent of coronary artery disease (Artaud-Wild, S.M., Connor, S.L., Sexton, G., Connor, W.E. (1993) Circulation 88: 2771-2779). In effect, the increased blood cholesterol concentration in humans seems to have a direct positive correlation with coronary heart disease. Therefore patients with coronary heart disease (CHD) or hypercholesterolemia are commonly recommended to decrease their dietary cholesterol intake.

Other foodstuffs of animal origin such as eggs, which are commonly used in the preparation of a variety of food products, present the same problem. Because of the special organoleptic traits of milk and eggs, it is difficult to replace them by other products with less cholesterol content.

The general awareness of the risks associated with high blood cholesterol levels is an important factor limiting the consumption of food substances that have high cholesterol content by a health-conscious public. In the past years it has been a significant health trend away from red meat, milk and eggs. Accordingly, there is a continuing and real interest in decreasing the intake of food substances that have high cholesterol content.

To address these problems, there is a need for methods to produce low-cholesterol versions of normally high-cholesterol foodstuffs, such as whole milk and eggs. Such methods should preferably not appreciably change the physical and organoleptic properties of the foodstuffs. The nutritional value of the treated foodstuffs should be preferably maintained, especially the levels of those components that are lipid-soluble and that are important for human nutrition (e.g., vitamins A and D, and essential fatty acids). Thus, the food treatment methods should yield products with lower cholesterol content but which are otherwise similar to the untreated foodstuffs. Additionally, the novel methods should preferably not require expensive equipment and materials or potentially toxic materials, such as organic solvents.

A number of methods have been described in patents in the US and other countries for reducing the cholesterol content of foodstuffs. For example, cholesterol can be removed from foodstuffs by the use of physicochemical methods. For instance, the use of supercritical fluids to produce liquid egg having reduced cholesterol content has been proposed (Ogasahara et al, US Patent 5,116,628, 1992). However, the high temperatures and pressures needed for the process can denature proteins present in the foodstuffs. Likewise, the production of low cholesterol butter oil by vapor sparging (Conte et al, 1992, US Patent 5,092,964) is another example of a method, which, due to the extreme conditions used, is likely to denature proteins and alter organoleptic properties of the foodstuffs.

The use of organic solvents to extract cholesterol from foodstuffs has also been proposed. Thus, Fallis et al (1978, US Patent 4,104,286) have proposed the use of aqueous ethanol, saponification, and extraction with hydrocarbons and methanol to obtain free cholesterol, saponified fats and edible egg powder. This process uses extreme conditions and large quantities of organic solvents that may contaminate the processed foodstuffs. Extraction with liquid dimethylether (Yano et al, 1980, US Patent 4,234,619) is similarly inconvenient and does not appear to be selective for cholesterol as other neutral lipids are removed from the foodstuff. Johnson et al. (1991, US Patent No. 4,997,668) applied solvent extraction to milk, but again the method does not appear to be selective for cholesterol and utilizes organic solvents that may contaminate foodstuffs.

A variation on the use of organic solvents is to employ oils to extract cholesterol from either aqueous or dry foodstuffs, like egg yolk and dairy products. (Bracco et al, 1982, US Patent 4,333,959; Keen, 1991, US Patent 5,039,541; Conte et al, 1992, US Patent 5,091,203; Merchant et al, 1995, US Patent 5,378,487; Jackeschky, 1998, US Patent 5,780,095). Again, these methods do not selectively extract cholesterol and oils contaminated with cholesterol are inevitably produced, which is undesirable.

Removal of cholesterol by formation of complexes with cyclodextrins has also been proposed for fatty substances of animal origin (Courregelongue et al, 1989, US Patent 4,880,573) and specifically in the case of dairy products (Chung Dae-Won, 1999, Foreign Patent WO 9917620). The formation of complexes of cholesterol and saponin has also been described as a means to reduce cholesterol in milk (Richardson, 1994, US Patent 5,326,579). These methods are, however, too expensive for industrial applications.

A different approach is based on the use of enzymes that modify cholesterol. Thus, the use of cholesterol reductases, that modify cholesterol into poorly absorbed sterols, has been proposed (Beitz et al, 1990, US Patent 4,921,710; Ambrosius et al, 1999, US Patent 5,856,156). Another proposed enzymatic approach is the conversion of cholesterol into epicholesterol, which is then further modified by an epicholesterol dehydrogenase (Saito et al, 1999, US Patent 5,876,993). These methods have the disadvantage that they do not result in the conversion of cholesterol into useful compounds for human nutrition.

There is therefore a need for methods for treating foodstuffs to reduce the amount of cholesterol. Preferably, the cholesterol is converted to one or more substances that are useful for human nutrition.

When *Tetrahymena* is grown in the presence of exogenous sterols, the biosynthesis of tetrahymanol is completely inhibited and the added sterol is accumulated by the organism and, in most cases, metabolized to other sterols. Different types of biotransformations have been observed, including $\Delta 7$ and $\Delta 22$ desaturation and the removal of ethyl, but not methyl groups, from C24 (Mallory, F.B. and Conner, R.L. (1971) Lipids 6:149-153; Conner, R.L., Landrey, J.R., Joseph, J.M., Nes, W.R. (1978) Lipids 13: 692-696; Ferguson, K.A., Davis, F.M., Conner, R.L., Landrey, J.R. and Mallory, F.B. (1975) J Biol Chem 250:6998-7005).

In the case of cholesterol, *Tetrahymena* transforms this sterol into the desaturated derivatives: $\Delta 7,22$-bis-dehydrocholesterol (a close analog of ergosterol, also called provitamin D2), $\Delta 22$-dehydrocholesterol and $\Delta 7$-dehydrocholesterol (provitamin D3). (Conner, R.L., Mallory, F.B., Landrey, J.R. and Iyengar, C.W.L. (1969), J Biol Chem 244:2325-2333). The desaturation of cholesterol at position 7 converts it into provitamin D3 and pro vit D derivatives, which upon UV irradiation in the skin can be activated to vitamin D. This biotransformation has seldom been observed in nature.

In another hand, vitamin D3, also named Cholecalciferol, is commonly prepared, in commercial practice, by irradiation of 7- dehydrocholesterol with ultraviolet light. Upon irradiation, intermediate products are formed (pre vitamin D3) which are converted into vitamin D3 by thermal rearrangement.

The precursor 7- dehydrocholesterol may be obtained via organic solvent extraction of animal skin (cow, pig, sheep) followed by extensive purification and crystallization.

Industrially, $\Delta 7$ dehydrocholesterol is commonly synthesized from cholesterol via a laborious chemical synthesis entailing the introduction of a double bond in the seven position of cholesterol. However, the introduction of this double bond in the seven position of cholesterol has several associated problems. (Gui-Dong Zhu and William Okamura, 1995, Chemical Reviews 95: 1877-1952).

The usual methods of synthesis from compounds having a 5-steroid structure, consist in the classical bromination/dehydrobromination procedure using 1,3 dibromo-5,5 dimethylhydantoin or N-bromosuccinimide. The resulting epimeric mixture of C-7 bromides is treated with trimethyl phosphite in xylene or collidine, yielding about 40 % of the desired 5,7 diene, along with a substantial quantity of the undesired 4,6-diene isomer (25%).

The unwanted contaminant, delta 4,6 diene, has plagued this conversion despite many decades of extensive studies, and several improvements for this process have been reported. (Confalone c.s. in J. Org. Chem. 1981, 46, 1030-32; German Patent Application No. 2547199).

For instance, the dehydrobromination of the 7-alfa-bromide of the acetate of cholesterol with $Bu_4NF.H_2O$ in THF or DMF was reported to give selectively the desired delta 5,7 diene. In this two-step procedure, provitamin D synthesis was achieved in 62 % yield with 95 % purity. Another possible alternative is the palladium catalyzed elimination of allylic esters, which yields 90 % of the desired homoannular diene.

Because the introduction of the delta 7,8 double bond is cumbersome, another approach has been to generate the desired 5,7 diene through base-catalyzed deconjugation of the easily available 1,4,6 trien-3-one. This reaction is followed by succesive reduction, diene protection and epoxidation. This simple route is hampered, however, by the low selectivity of the epoxidation step.

In conclusion, although many solutions have been proposed for improving the chemical synthesis of provitamin D, the great number of reaction steps and the complexity of the synthesis constitute a serious disadvantage. Moreover, yields in these syntheses are usually poor, chemicals needed for carrying out the procedures may have to be specially prepared, and in some cases also special equipment is required. Selective introduction of a double bond on the seven position of a delta 5 sterol is also important for the synthesis of valuables intermediates for important products, i,e., in the preparation of $1\alpha$ $25(OH)_2$ vitamin D3 analogs.

## SUMMARY OF THE INVENTION

The primary object of the present invention is to provide a cell free extract comprise at least one sterol desaturase that catalyze desaturation of sterols to a more desaturated derivate (e.g. $\Delta$7-dehydrocholesterol, $\Delta$22-dehydrocholesterol or $\Delta$7-22 bisdehydrocholesterol. The cell free extract can be a homogenate, a microsomal fraction, an enriched- desaturase fraction, substantial pure $\Delta$7 cholesterol desaturase enzyme or substantial pure $\Delta$22 cholesterol desaturase enzyme obtained from any Ciliate phylum microorganism as *Tetrahymenidae* family.

The present invention provides a substantial pure $\Delta$7 cholesterol desaturase enzyme from ciliates, more particularly from *Tetrahymenidae* family microorganism, wherein said enzyme is capable of catalyzing the conversion of a sterol substrate in $\Delta$7 dehydroesterol by introducing a double bond at the position seven in the sterol molecule.

According to the invention, the substantial pure $\Delta$7 cholesterol desaturase enzyme is characterized by

(a) having a molecular weight of approximately 60 kDa by gel chromatography;
(b) having an optimum pH range for enzymatic activity between 6.5-8.5;
(c) having an optimum temperature range for enzymatic activity of 28°C to 35°C;
(d) being unaffected by metal ions such as $Ca^{+2}$, $Mn^{+2}$ and $Mg^{+2}$ ,EDTA concentrations and 2-mercaptoethanol;
(e) being inactivated after 1 minute at 100°C;
(f) being storage at -20°C by at least 6 months.

It is another object of the present invention to provides a substantial pure $\Delta$22 cholesterol desaturase enzyme from ciliates, more particularly from *Tetrahymenidae* family microorganism, wherein said enzyme is capable of catalyzing the conversion of a sterol containing substrate in $\Delta$22 dehydrosterol by introducing a double bond at the position twenty-two in the sterol molecule.

According to the invention, the substantial pure $\Delta$22 cholesterol desaturase enzyme is characterized by

(a) having a molecular weight of approximately 60 kDa by gel chromatography;
(b) having an optimum pH range for enzymatic activity between 5.5-8.5;
(c) having an optimum temperature range for enzymatic activity of 28°C to 35°C;
(d) being unaffected by metal ions such as $Ca^{+2}$, $Mn^{+2}$ and $Mg^{+2}$ and EDTA concentrations;
(e) being inactivated after 1 minute at 100°C;
(f) being storage at -20°C by at least 6 months.

It is still a further object of the present invention to provide a process for preparing a substantial pure $\Delta$7 cholesterol desaturase and a substantial pure $\Delta$22 cholesterol desaturase enzymes from ciliates, more specifically from *Tetrahymenidae* family, said process comprising the steps of:

(a) culturing a microorganism in a suitable medium, wherein said microorganism is capable of producing $\Delta$7 cholesterol desaturase and/or $\Delta$22 cholesterol desaturase enzymes;
(b) Disintegrating the culture and extracting the same with buffer solution containing, if necessary, non-ionic surfactant or glycerol as stabilizer;
(c) subjecting the extract to a chromatography purification under suitable conditions; and
(d) eluting and recovering said $\Delta$7 cholesterol desaturase enzymes or $\Delta$22 cholesterol desaturase enzymes.

The present invention provides methods for reducing the amount of cholesterol in cholesterol-containing foodstuffs, using cell free extracts from Ciliates as *Tetrahymena,* preferably without significantly altering the organoleptic properties of the treated cholesterol- containing foodstuffs, such as dairy products, such as milk, egg and other products of animal origin.

Said methods comprising at least the step of treating a cholesterol-containing foodstuff by incubate the same with a cell free extract obtained from a *Tetrahymenidae* family microorganism in a effective amount to enable the following changes in the composition of the foodstuff: (a)reduce the level of cholesterol and (b) increase the level of at least one cholesterol desaturated derivative: $\Delta$7-dehydrocholesterol, $\Delta$22- dehydrocholesterol or $\Delta$7-22 bisdehydrocholesterol.

It is another object of the present invention to provide methods for increasing the amount of $\Delta$7-dehydrosterol, $\Delta$22- dehydrosterol and $\Delta$7-22 bis dehydrosterol in foodstuffs. Therefore, the present invention provides a method for increasing the amount of provitamin D in foodstuffs. Examples of vitamin D precursors are: $\Delta$7-dehydrocholesterol (provitamin D3) and $\Delta$7-22didehydrocholesterol (a close analog of ergosterol, provitamin D2)

Any foodstuff of animal origin can be treated in accordance with the methods of the present invention. The foodstuff can be a dairy product, such as milk, egg yolk or an egg product. The milk can be milk from any mammal, such as cow or goat, and egg yolk can be from eggs of any species of bird, such as domestic chicken eggs. Representative examples of other foodstuffs that can be treated by the methods of the present invention are broth, yogurt, cheese, cream and soups that contain material of animal origin including cholesterol.

When the methods of the present invention are utilized to reduce the level of cholesterol in milk, the level of cholesterol in the milk after treatment is preferably less than 90%, more preferably less than 70%, and more preferably less than 30% of the level of cholesterol in the milk before treatment.

In one embodiment of the invention the cell free homogenate fraction, microsomal fraction and/or desaturase-enriched fraction obtained from the culture of a member of the family *Tetrahymenidae* is incubated under conditions that enable a reduction in the level of cholesterol in the treated foodstuff and an increase in the level of at least one cholesterol desaturated derivative, such as Δ22- dehydrocholesterol or a vitamin D precursor (formed by the biochemical conversion of the cholesterol). Representative examples of vitamin D precursors are Δ7-dehydrocholesterol and Δ7,22-didehydrocholesterol.

Representative incubation conditions for lowering the level of cholesterol and increasing the cholesterol desaturated derivatives in liquid suspensions such as milk or diluted egg yolk utilizing *Tetrahymena termophila* cell free extract are: incubation temperature of from 24 °C to 37 ° C, at a pH of between 5.0 and 8.0, for a time period of from 1 hour to 24 hours. For example, the methods of the present invention can be used to decrease between 10% to 90% the level of cholesterol initially present in foodstuff to more desaturated cholesterol derivatives such as: Δ7-dehydrocholesterol, Δ22-dehydrocholesterol and Δ7-22 bisdehydrocholesterol. Typically, about 90% of the cholesterol originally present in foodstuff is recovered as desaturated cholesterol derived after treatment in accordance with the present invention.

The methods of the present invention provide several advantages. For example, the methods not only selectively decrease foodstuff cholesterol concentration, preferably without significantly altering organoleptic properties, but at the same time, the process of desaturating cholesterol at position 7, converts it into Δ7-dehydrocholesterol, also known as provitamin D3. Thus, a single reaction decreases an unwanted material (cholesterol), converting it into a desirable one (provitamin D3).

The methods of the present invention make unnecessary milk supplementation with vitamin D by providing provitamin D in amounts that are sufficient for daily requirements. In addition, while vitamin D (a supplement normally added to milk), can have toxic effects, the provitamins have a much lower risk of toxicity. This is due to the fact that the provitamins lack vitamin D activity and they have to undergo chemical conversion by exposure to UV light to become active vitamins.

It is still another object of the present invention to provide a process for manufacturing Δ7 dehydrosterols, Δ22 dehydrosterols and/or Δ7,22 dehydrosterols comprising:

(a) mixing a cell free extract of the invention with a sterol containing substrate;
(b) incubating the mixture for a period of time enough to produce Δ7 dehydrosterol and/or Δ22 dehydrosterol, and/or Δ7,22 bis dehydrosterol;
(c) recovering said Δ7 dehydrosterol and/or Δ22 dehydrosterols and/or Δ7,22 bis dehydrosterol by solvent extraction and chromatographic purification.

It is further object of the present invention to provide compounds obtained by the method of the present invention which compounds constitute high value added compounds as they are synthetic intermediates in the preparation of vitamin D or vitamin D analogs, useful in the pharmaceutical industry.

## BRIEF DESCRIPTION OF THE DRAWINGS

The foregoing aspects and many of the attendant advantages of this invention will become more readily appreciated as the same becomes better understood by reference to the following detailed description, when taken in conjunction with the accompanying drawings, wherein:

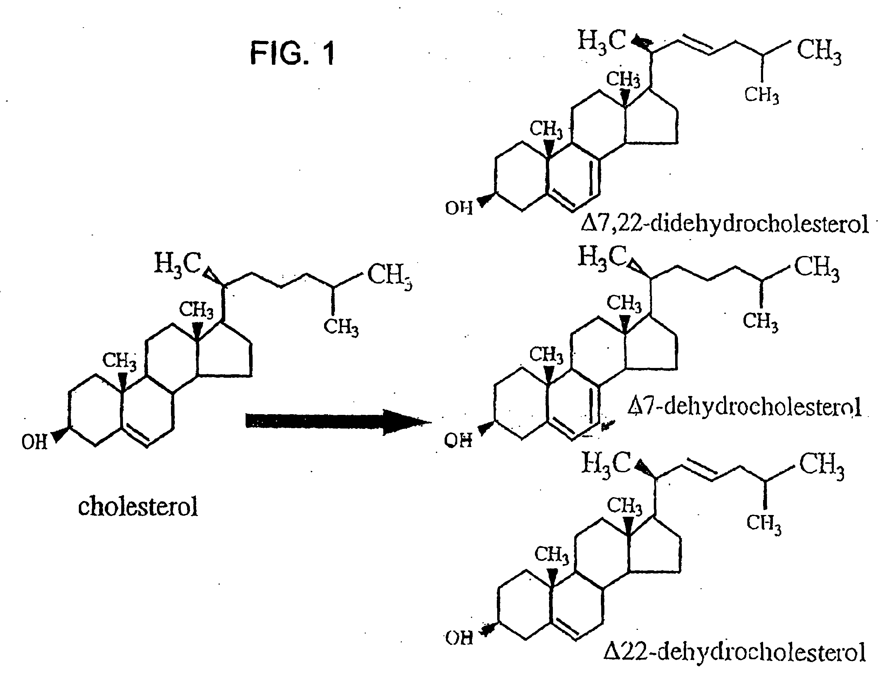

FIGURE 1 shows three desaturation products of cholesterol.

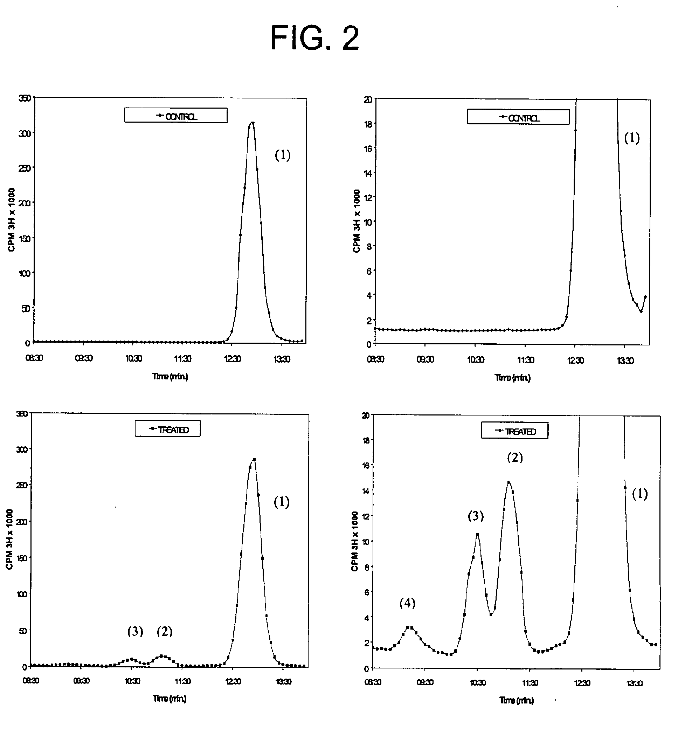

FIGURE 2 shows high performance liquid chromatograms showing the separation of radiolabeled cholesterol (peak 1) from its desaturated derivatives, Δ7- dehydrocholesterol (peak 2), Δ22- dehydrocholesterol (peak 3) and Δ7,22-didehydrocholesterol (peak4) before (control) and after (treated) incubation with a microsomal fraction of *Tetrahymena* cells. The unsaponifiable fraction was extracted with chloroform and subjected to HPLC equipped with a radioactivity detector. Further details are described in Example 1.

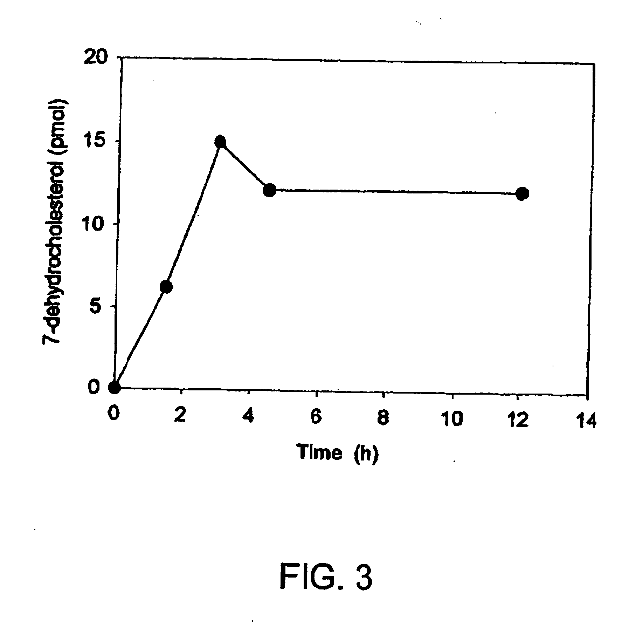

FIGURE 3 shows the variation of Δ7-dehydrocholesterol desaturase activity of *Tetrahymena thermophila* microsomal fraction with incubation time. Values are mean of three parallel incubations. Standard deviation did not exceed 5%.

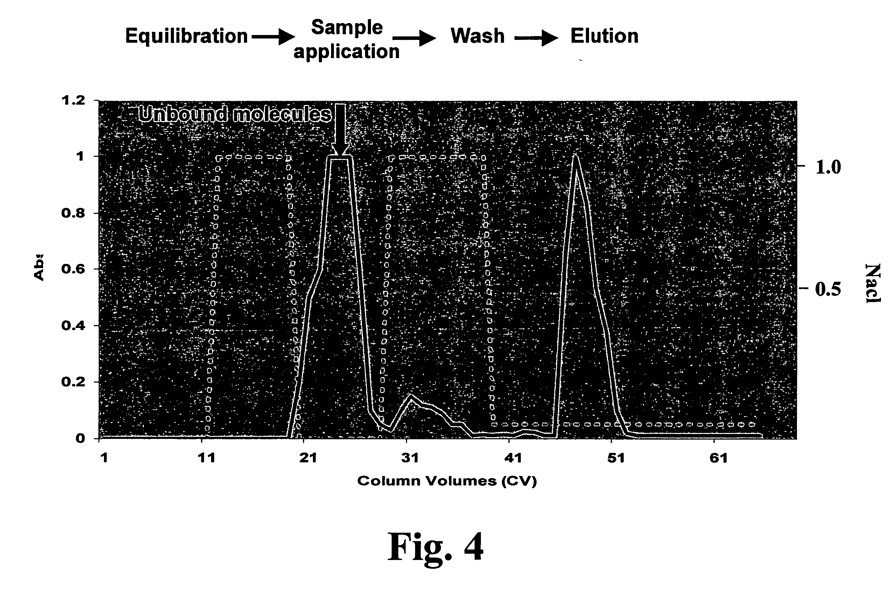

FIGURE 4 shows a chromatographic diagram corresponding to HIC of purification of $\Delta 7$ cholesterol desaturase. The solubized fraction from *Tetrahymena* membranes was applied to a Hydrophobic Interaction Chromatography column equilibrated and eluted. Unbonded protein fraction was collected and further purified by Ion Exchange Chromatography

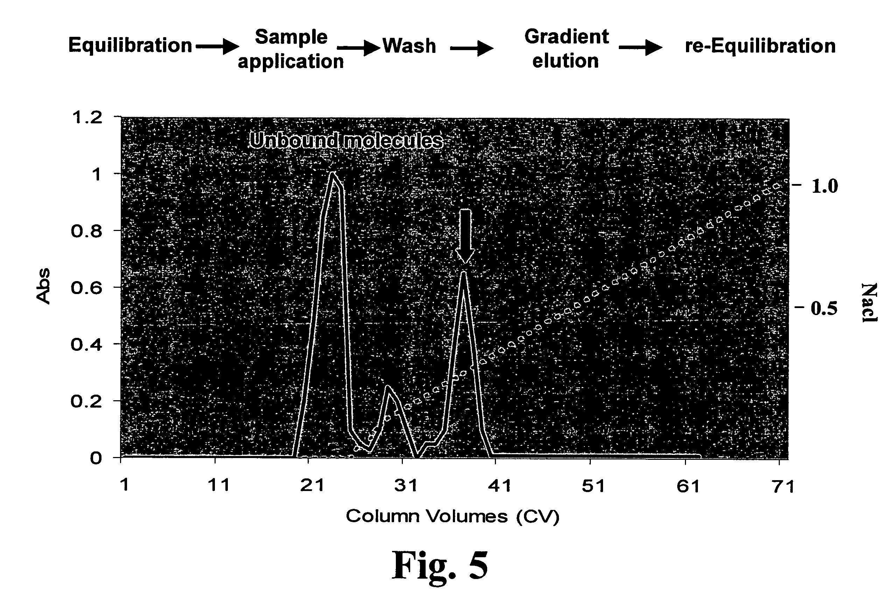

FIGURE 5 shows a chromatographic diagram corresponding to ionic exchange of purification of $\Delta 7$ cholesterol desaturase. The $\Delta 7$ cholesterol desaturase obtained from H.I.C was applied to a SP Sepharose HP column and eluted. The fraction was collected. Active fraction, indicated by an arrow, were pooled and further purified by Gel Filtration Chromatography.

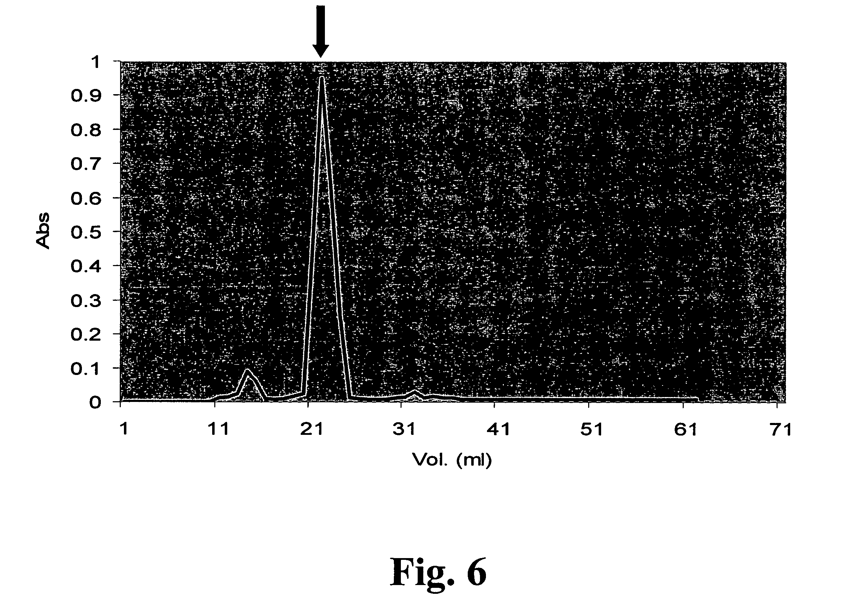

FIGURE 6 shows a chromatographic diagram corresponding to molecular exclusion of purification of $\Delta 7$ cholesterol desaturase. The $\Delta 7$ cholesterol desaturase obtained from the Ion Exchange step was applied to Suprose column and eluted. Fraction of 1.0 ml was collected and $\Delta 7$ cholesterol desaturase activity was assayed.

## DETAILED DESCRIPTION OF THE PREFERRED EMBODIMENT

As used herein, the term "*cholesterol-containing foodstuff*" means any natural or synthetic substance that contains cholesterol and that can be consumed by a mammal to provide nutrients.

As used herein, the term *"milk product"* means milk obtained from the female of any mammalian species, and any edible product or component derived from milk. Representative examples of milk product include whole milk, milk supplemented with one or more nutrients (such as vitamins and minerals), low-fat and skimmed milk, yogur, and cream.

As used herein, the term *"cell free extract"* means any extract obtained from cultured organisms in which no living cells are detected. Examples of cell free extracts comprise: (a) homogenate fraction obtained by cell lysis of the organism, (b) microsomal fractions, including the membranes fraction present in the homogenate fraction (c) desaturase enriched fraction, obtained by extraction, precipitation and/or purification of the proteins present in the microsomal preparation and substantial pure $\Delta 7$ cholesterol desaturases enzymes and substantial pure $\Delta 22$ cholesterol desaturases enzymes obtained by chromatography techniques.

As used herein, the term "sterol" comprises an aqueous suspension of sterols, an aqueous micelle solution of sterols, an aqueous solution in which sterols containing organic solvent layer is emulsified. For the purpose of description of this invention the sterols molecules are such as cholesterol. The sterol used in the present invention as a substrate includes various sterols, their C-3 ester or ether derivatives and the intermediates formed in the oxidation thereof. Sterols posses a hydroxy group at C1, C-3, and /or in the side chain (e.g. C24, C25), normally a double bond at C-5, a side chain of 8 to 10 carbon atoms at C-17, and in some cases, a double bond at C-7, C-8, C-9 (11), or in the side chain or like of the perhydrocyclopentanophenanthrene nucleous, although the cyclopentano phenanthrene nucleous may be saturated. Example of such sterols are cholesterol, 1-alpha hydroxycholesterol, 25 hydroxycholesterol, 1-alpha 25 dihydroxycholesterol, 24 hydroxycholesterol. Especially prefers is cholesterol.

As used herein, the term "cholesterol-containing product" comprises any animal product that may provide a rich source of cholesterol, i.e., skin, feathers, eggs, etc.

It is well known for any person skilled in the art that $\Delta 7$ cholesterol desaturase and $\Delta 22$ cholesterol desaturase enzymes are capable of establishing a double bond, at position 7 or 22, respectively, in any sterol molecule.

*Preparation of a protozoan culture:* any suitable growth medium can be used for culturing the cells to be used in the preparation of cell free extracts. For example, media based on yeast extract, glucose and skimmed milk can yield high cell densities at very low costs (Kiy, T. and Tiedtke, A. (1992), Appl. Microbiol. Biotechnol. 37:576-579, incorporated herein by reference). It is possible also to use a modification of the medium described by Kiy and Tiedtke (1992). The composition of this medium includes yeast extract (0.1 to 10% w/v, preferably 0.5% w/v), glucose (0.1 to 5% w/v, preferably 1% w/v), iron citrate (0.0001 to 0.1% w/v, preferably 0.003% w/v), supplemented with between 1 and 50% v/v fluid skimmed milk (preferably between 5 and 20% v/v). Another useful medium for culturing the protozoa has the same composition as the foregoing, Kiy and Tiedtke medium, except that the fluid milk was replaced with between 0.1 and 5% (preferably 1%) w/v tryptic casein hydrolysate and cholesterol (0.2 mg/100ml).

Cultivation of the protozoa useful in the practice of the invention can be achieved by any suitable means. For example, fermentors that utilize mechanical stirring, or air lift fermentors can be utilized for large-scale cultivation of the protozoa useful in the practice of the invention.

The optimal culture temperature depends on the strain of protozoa, but typically falls within the range of from 20°C to 38°C. Similarly, the optimal culture pH depends on the strain of protozoa, but typically falls within the range of from pH 5.0 to pH 8.0. For example, a pH of 6.8 has been successfully used to cultivate *Tetrahymena thermophila* wild type strain CU399. Culture time also depends on the protozoan strain and the chosen method of cultivation. Typically, culture times of between 10 and 80 hours (such as 23-25 hours) are adequate.

Cultivation of the protozoa can be carried out with or without agitation. When agitation is used, exemplary rates of stirring are in the range of from 50 rpm to 600 rpm, typically about 100 rpm. Finally, to achieve high protozoan densities in a relatively short time, cultivation can be carried out in airlift bioreactors (Hellenbroich, D., et al., Appl. Microbiol. Biotechnol. 51:447-455 (1999), incorporated herein as reference) utilizing any suitable medium, such as the media disclosed in Tiedtke and Kiy (German Patent DE 4238842); Kiy and Tiedtke, 1992, supra, each of which publications are incorporated herein as reference. Typically, cell densities of up to $2 \times 10^7$ cells/ml can be achieved.

A preferred embodiment of the invention is based upon the discovery that, by a very special manipulation of the sterol composition in the culture medium, the cholesterol desaturase content of the microorganisms is greatly augmented. Furthermore, selective provitamin D or analogs is obtained

Table 1 shows the effect of different growth conditions on desaturases productivity in *Tetrahymena* strain CU399. The activity is expressed as percentage of added cholesterol transformed by the solubilized fraction.

Table 1

| Sterol composition of the medium | $\Delta$ 7 desaturase activity | $\Delta$ 22 desaturase activity |
|---|---|---|
| Without sterols | - | - |
| Add 1 mg/100 ml of cholesterol | 2% | 30% |
| Add 1 mg/100 ml of 22,dehydrocholesterol | 25% | 1% |

For commercial production of cholesterol desaturase the gene or genes for cholesterol desaturase may be transferred into another microorganism such as E. *coli.* The process would be similar to that now in use for making human insulin, bovine growth hormone, and porcine growth hormone.

Any microorganism including variants and mutants can be used for the production of the cholesterol desaturases, so long as it belongs to the genus *Tetrahymena, Paramecium* and *Colpidium,* and is capable of producing cholesterol desaturases. A specific example of such microorganism is *Tetrahymena* CU399 and can be obtained from public depositories such as the American Type Culture Collection (ATCC), 10801 University Boulevard, Manassas, VA 20110-2209, U.S.A.

It will be apparent to any person skilled in the art that ciliate organism other than the *Tetrahymena* thermophyla may be employed for obtaining 7 cholesterol desaturase and 22 cholesterol desaturase enzymes. Such other ciliates are, for example: *Tetrahymenidae as T. pyriformis, T. patula, T. rostrata, T. vorax, T. paravorax, T. chironomi, T. setifera, T. corlissi, T. stegomyae and T. limacis*, and also from other family members of class Oligohymenophorea as *Colpidium*; and from members of class Nassophorea, such as *Paramecium*.

*Preparation of cell free extracts:* In the first step of the concentration process of this invention a homogenate fraction is prepared. The ciliates were harvested from late-log or stationary phase cultures, from of 24 hours to 40 hours culture, removed by centrifugation and suspended 1/10 in 0.2 M $Na_2HPO_4$ buffer (pH 7.2), washed once in the same buffer such as an isotonic aqueous-based buffered salt solution and finally suspended in 10 mM Tris-HCl, 250 mM sucrose, 5 mM $MgCl_2$, 1 mM EDTA (TES-Mg buffer). The buffer has a pH in the range 6.0 to about 7.5. Homogenates were prepared by sonication in an ice-water bath until complete cell lysis. The homogenate was centrifuged and the supernatant ("homogenate fraction") was tested for cholesterol desaturase activity.

The microsomal fraction was prepared by centrifugation at 105,000 xg for 1.5 hours of the homogenate. The supernatant was discarded and the pellet obtained was suspended in the same buffer TES-Mg. All procedures were carried out at 4°C. Both pellet and supernatant were tested for desaturase activity.

The desaturase-enriched fraction may be isolated from the microsomal fraction thus obtained, in a conventional manner. The protein fraction, for example, can be obtained by extracting the microsomal fraction with a detergent solution, centrifugation and further treating the supernatants with ammonium sulphate. After centrifugation the precipitate is dialysed to eliminate salts. This solution contains the desaturase- enriched fraction.

Further purification may be achieved by any known method including but not limited to ion exchange chromatography, hydrophobic interaction chromatography, size exclusion chromatography or affinity chromatography, as it be later disclosed.

The concentrate can be further treated to obtain substantially (100%) pure $\Delta$7 dehydrocholesterol desaturase enzyme and $\Delta$22 dehydrocholesterol desaturase enzyme, however the cell free fractions itself may be used for the subsequently described methods of cholesterol transformation to $\Delta$7 dehydrocholesterol, $\Delta$22 dehydrocholesterol or $\Delta$7,22 bis- dehydrocholesterol.

If desired, foodstuffs (for example, milk and egg yolk) treated in accordance with the present invention can be dehydrated and used as a powder.

*Cholesterol desaturase activity of a cell free extract:* **homogenate, microsomes or desaturase- enriched fractions were tested by adding radiolabeled cholesterol, and various cofactors such as ATP, NAD, NADP or their reduced derivatives. After incubation at about 30°C during 1 to 6 hours, the reaction was stopped and the sterols were extracted from the reaction mixture and analised as described in Example 1.**

The radioactive sterols present in the mixture were identified as cholesterol, Δ7-dehydrocholesterol, Δ22-dehydrocholesterol and Δ7-22didehydrocholesterol by co-migration in different chromatographic systems and mass spectroscopy analysis.

Practically all the radioactivity was associated with either untransformed cholesterol or desaturated derivates present in various amounts in the isolated fractions.

Essentially the same procedure was used for testing cholesterol desaturase activity in all the cell free extracts including homogenates, microsomal fractions and desaturase-enriched fractions.

As an example, Table 2 describes the cholesterol derivatives formed after incubation of homogenates fractions with $^3$H- cholesterol.

Table 2.

| Cholesterol derivatives formed by incubation of cellular fractions from *T. thermo- phila* with radiolabeled cholesterol | | | |
|---|---|---|---|
| **Cellular Fraction** | % of total radioactivity converted to $\Delta^7$ dehydrocholesterol | % of total radioactivity converted to $\Delta^{22}$ dehydrocholesterol | % of total radioactivity converted to $\Delta^{7-22}$ bisdehydrocholesterol |
| Homogenate | 7.5 ± (1.1) | 5.2 ± (0.8) | 3.3 ± (0.2) |
| Microsomal fraction | 9.3 ± (1.7) | 7.1 ± (1.8) | 1.9 ± (0.7) |
| Supernatant from microsomal preparation | N. D.[c] | N. D.[c] | N. D.[c] |

c: ND: not detected

If necessary, coenzymes such as NAD(H), NADP(H) may be added to the enzymes reaction.

The conversion of cholesterol increased lineally up to 3 hours of incubation. After this period, no further conversion was observed (Figure 3). Addition of ATP significantly increased the desaturase activity.

In addition, Δ7 cholesterol- desaturase activity was completely abolished after boiling during 10 min at 100° C. This latter condition was therefore used as a control.

The isolation and characterization of Δ7- cholesterol desaturase activity has been published and is included herein as a reference (Valcarce et al., Appl. Microbiol. Biotecnol. 53: 591-595, 2000).

*Purification and Characterization of Δ7 cholesterol desaturases:*

A number of conditions were assayed for improving enzyme yields and for optimizing the activities measurements.

A significant increase in the desaturase activity (5 to 10 fold) was observed when *Tetrahymena* is grown in PPYE medium plus Δ22 dehydrocholesterol.

The Δ7 cholesterol desaturase from *Tetrahymena* of the present invention is a novel enzyme, which catalyze the reaction of cholesterol in accordance with the following equation:

$$\text{Cholesterol + NAD(P)H} \rightarrow 7 \text{ dehydrocholesterol + NAD(P)}$$

The activity of the enzyme can easily be determined through the use of the above reaction, for example by determining the 7 dehydrocholesterol formed using a classical RP-HPLC method.

The physicochemical properties of the Δ7 cholesterol desaturase are as follows:

*Optimal pH:* The desaturase activity show optimal conversion rate at pH 7.4. Beyond the pH limits of 5 and 9.5, the desaturase is not active.

*Temperature stability*: optimal desaturase activity is obtained when the enzyme is incubated at temperatures from 30 to 35 °C. Desaturase activity is sub optimal at temperatures below 25°C and decrease when the enzyme incubation is carried out at temperatures upper than 42°C.

*Detergent inhibition:* the desaturase activity is affected by various detergents such as: triton X100 and X114, Tween 80 and deoxycholate. At concentration near to critical micellar concentration Octylthyoglucoside (OTG) effectively solubilize the enzyme and this concentration is only partially inhibitory for the enzyme.

*Molecular weight:* apparent molecular mass of the purified Δ7 cholesterol desaturase is estimated to be 60 kDa by gel filtration chromatography.

*Ions and quelators effect:* the desaturase rate is not influenced by EDTA concentration suggesting that the enzyme does not require divalent metals cations for activity. Effectively, the activity is not affected by $Ca^{+2}$, $Mn^{+2}$ and $Mg^{+2}$.

*Storage conditions:* Solubilized enzyme could be stored at -80 °C and -20 °C for at least 6 months with small change in enzymatic activity. The addition of 20% of glycerol stabilized the activity. Lyophilization could also be used for storage, the use of 1 mg/ml of BSA showed optimal performance.

*Specific activity*: the rate of reaction of the purified enzyme is linear with respect to the enzyme protein content up to 5 mg/ml and the incubation time within 60 min.

*Inhibitors:* The activity was inhibited by the addition of Δ22 dehydrocholesterol to the reaction media.

The purification of Δ7 cholesterol desaturase may be carried out by employing any purification method known in the art, according to a preferred embodiment, however, the purification of Δ7 cholesterol desaturase is carried out as it is disclosed in Example 5. Following Table 3 shows the results of the specific activity and yielding when the purification method disclosed in Example 5 is employed.

Table 3

| Purification step | Total protein (mg) | Total activity (nmol) | Specific activity (nmol/mg protein x min) | Yield (%) |
|---|---|---|---|---|
| *Microsomal fraction* | 5000 | 66000 | 0.11 | 100 |
| *Soluble fraction (OTG)* | 2800 | 43680 | 0.13 | 66 |
| *Salt precipitation* | 2200 | 39600 | 0.15 | 60 |
| *HIC* | 272 | 34320 | 1.05 | 52 |
| *IEC* | 13.7 | 11880 | 7.20 | 18 |
| *SEC* | 2.9 | 5280 | 15.1 | 8 |

Figures 4, 5 and 6 show the chromatographic diagrams of purification of Δ7 cholesterol desaturase by a hydrophobic interaction chromatography, a cationic exchange chromatography and a molecular exclusion chromatography, respectively.

*Characterization and Purification of Δ22 cholesterol desaturases:*

The Δ22 cholesterol desaturase enzyme of the present invention is a novel enzyme, which catalyzes the reaction of cholesterol in accordance with the following equation:

$$Cholesterol + NAD(p)H \rightarrow 22\ dehydrocholesterol + NAD(P)$$

The activity of the enzyme can easily be determined through the use of the above reaction, for example by determining the Δ22 dehydrocholesterol formed using a classical RP-HPLC method.

The physicochemical properties of Δ22 cholesterol desaturase are as follows: *Optimal pH:* The desaturase activity show optimal conversion rate at pH 7.4. Beyond the pH limits of 5 and 9.5, the desaturase is not active.

*Temperature stability*: optimal desaturase activity was obtained when the enzyme is incubated at temperatures from 30 to 35 °C. Desaturase activity is sub optimal at temperatures below 25°C and decrease when the enzyme incubation is carried out at temperatures upper than 42°C.

*Detergent inhibition:* the desaturase activity is affected by various detergents such as: triton X100 and X114, Tween 80 and deoxycholate. At concentration near to critical micellar concentration Octylthyoglucoside (OTG) effectively solubilize the enzyme and this concentration is only partially inhibitory for the enzyme.

*Molecular weight:* apparent molecular mass of the purified Δ22 cholesterol desaturase is estimated to be 60 kDa by gel filtration chromatography.

*Ions and quelators effect:* the desaturase rate is not influenced by EDTA concentration suggesting that the enzyme does not require divalent metals cations for activity. Effectively, the activity is not affected by $Ca^{+2}$, $Mn^{+2}$ and $Mg^{+2}$.

*Storage conditions:* Solubilized enzyme could be stored at -80 °C and -20 °C for at least 6 months with small change in enzymatic activity. The addition of 20% of glycerol stabilized the activity. Lyophilization could also be used for storage, the use of 1 mg/ml of BSA showed optimal performance.

*Specific activity*: the reaction velocity of the purified enzyme is linear with respect to the enzyme protein content up to 5 mg/ml and the incubation time within 120 min.

*Inhibitors:* The activity was inhibited by 2-mercaptoethanol.

The purification of Δ22 cholesterol desaturase may be carried out by any purification method well known in the art; however, according to a preferred embodiment of the invention the purification of Δ22 cholesterol desaturase is carried out with the method disclosed in Example 6. Following Table 5 shows the results of the specific activity and yield when the purification method disclosed in Example 6 is employed.

Table 5

| Purification step | Total protein (mg) | Total activity (nmol) | Specific activity (nmol/mg protein x min) | Yield (%) |
|---|---|---|---|---|
| *Microsomal fraction* | 5000 | 85000 | 0.14 | 100 |
| *Soluble fraction (OTG)* | 2917 | 59500 | 0.17 | 70 |
| *Salt precipitation* | 2423 | 55250 | 0.19 | 65 |
| *IEC (anionic)* | 55.8 | 22100 | 3.3 | 26 |
| *SEC* | 4.8 | 9350 | 16.0 | 11 |

*Treatment of Foodstuffs:* The incubation of cholesterol containing foodstuffs with a homogenate, a microsomal fraction or an enriched- desaturase fraction is typically conducted at a temperature in the range of from 20°C to 40 °C. The pH of the foodstuff is adjusted to fall within the range of pH 6.0 to pH 8.0. The desired incubation time mainly depends on the kind and extent of dilution (if any) of the foodstuff being treated. Typically, incubation times are from 0.5 hours to 72 hours (preferentially from 1 to 7 hours).

The incubation of the cell free extracts such as homogenate, microsomal fraction or enriched-desaturase fraction with the foodstuff can be carried out with or without agitation. If stirred, typically a stirring speed of between 50 rpm and 300 rpm (such as about 150 rpm) is utilized. If necessary the foodstuff can be diluted for optimal enzymatic activity. Milk for instance can be diluted with water or any other diluent to a concentration ½ to 1/100.

Incubation of the foodstuffs with cell free extracts can be carried out in presence of ATP or other high-energy compounds.

The methods of the present invention can be used to convert between 5% and 90 % of the cholesterol initially present in the foodstuffs to cholesterol derivatives, such as Δ7-dehydrocholesterol (provitamin D3), Δ22-dehydrocholesterol and Δ7,22-didehydrocholesterol, a close analog of ergosterol (provitamin D2). Typically, about 90 % of the cholesterol originally present in the foodstuff is recovered as untransformed cholesterol or desaturated cholesterol after treatment of the foodstuff in accordance with the present invention.

Any foodstuff of animal origin can be treated in accordance with the methods of the present invention. Examples of these are yogur, cheese, cream, eggs, milk, either whole milk or supplemented with one or more nutrients (such as vitamins and minerals). For example, the egg yolk can be diluted 1/10 and incubated by the desaturase-enriched fraction for 3 hours at 30 °C, with gentle stirring (100rpm). Utilizing this method there was a decrease in the amount of cholesterol and an increase in the unsaturated derivatives.

*Treatment of cholesterol substrate by substantially purified Δ7 cholesterol desaturase enzyme:*

The substantially purified Δ7 cholesterol desaturase enzyme according to the invention is employed for obtaining Δ7 dehydrocholesterol (pro-vitamin D3), useful as an intermediate for obtaining Vitamin D according to methods well known in the art. Table 4 shows results from the cholesterol transformation into Δ7 dehydrocholesterol according to the method disclosed in Example 7. The reaction characteristics are listed in following Table 4.

Table 4

| Protein concentration | 1 mg/ml |
|---|---|
| *Specific activity (IEC fraction)* | 7 nmol/min x mg protein |
| *Reaction time* | 60 min |

Table 4   (continued)

| Protein concentration | 1 mg/ml |
|---|---|
| *Conversion yield* | 84% |
| *Initial cholesterol* | 0.5 mM |

By employing the method disclosed in Example 7 a high (84%) yield in the conversion of cholesterol into $\Delta 7$ dehydrocholesterol is obtained. Briefly, in one preferred embodiment of the invention, an enzymatic method for producing $\Delta 7$ dehydrocholesterol is provided which comprises the steps of: a) adding soluble cholesterol to the enzyme preparation, for example, selected from the group of: microsomal fraction, desaturase-enriched preparation containing $\Delta 7$ desaturase and preferably, substantial pure $\Delta 7$ desaturase; b) incubating for a time enough for producing a high yield (above 85%) of $\Delta 7$ dehydrocholesterol; c) extracting the sterols with a water immiscible solvent d) recovering pure 7 dehydrocholesterol.

This method provides several advantages. For example the method selectively transforms cholesterol to provitamin D3. During the process, no other isomers are formed in addition to 7 dehydrocholesterol, avoiding the complex problem associated with the purification of the product from sterol mixtures.

The method selectively transforms cholesterol from sterol mixtures avoiding the drawbacks associated with high purification of the starting material, and also has a high yield and avoid the use of toxic products and complex compounds used in the current chemical method of synthesis of 7 dehydrocholesterol.

*Treatment of cholesterol substrate by substantially purified $\Delta 7$ cholesterol desaturase and 22 cholesterol desaturase enzymes:*

Both pure desaturases enzymes of the present invention may be used in aqueous solutions or as a powder. Preferably, they are dissolved in water and the resulting aqueous solution is used in the different application. If necessary, surfactants such as OTG may be added to the solution. Other stabilizers such as polyols, glycerol, methanol can be used.

In accordance with the invention the substantial pure enzymes can also be used immobilized to a solid matrix. It is also in the scope of this invention the use of cholesterol desaturases (substantial pure $\Delta 7$ cholesterol desaturase, substantial pure $\Delta 22$ cholesterol desaturase, or a combination of both) immobilized in a solid matrix, such as alginate beads, for cholesterol biotransformation.

If necessary, coenzymes such as NAD(P), NAD(P)H may be added to the enzymes reactions.

The enzymes preparation (containing $\Delta 7$ cholesterol desaturase and $\Delta 22$ cholesterol desaturase) provides the obtention of another Vitamin D derivative, namely $\Delta 7,22$ bis-dehydrocholesterol by employing cholesterol as substrate.

It will be obvious for any person skilled in the art that the enzymes preparations (containing $\Delta 7$ cholesterol desaturase and/or $\Delta 22$ cholesterol desaturase) may be employed for obtaining several Vitamin D derivatives by employing cholesterol as substrate, with said cholesterol being in a pure form or forming part of several foods or other cholesterol sources, e.g. eggs, skin, feathers, milk, etc.

Table 6 shows the results of the transformation of cholesterol into $\Delta 7,22$ bis dehydrocholesterol according to the method disclosed in Example 8. The reaction characteristics are listed in following Table 6.

Table 6

| Protein concentration | 2 mg/ml |
|---|---|
| $\Delta 7$ *specific activity (IEC fraction)* | *3.5* nmol/min x mg protein |
| *$\Delta 22$ specific activity (IEC fraction)* | 3.3 nmol/min x mg protein |
| *Reaction time* | 60 min |
| *Conversion yield* | 84% |
| *Initial cholesterol* | 0.5 mM |

By employing the method disclosed in Example 8 a high (84%) yield in the conversion of cholesterol into $\Delta 7,22$ bis dehydrocholesterol is obtained. Briefly, in one preferred embodiment of the invention, an enzymatic method to produce $\Delta 7,22$ bis dehydrocholesterol is provided which comprise the steps of: a) adding soluble cholesterol to the $\Delta 7$ and $\Delta 22$ enzyme preparations, for example, selected from the group of: microsomal fraction, desaturase- enriched preparation containing Δ7 and Δ22 desaturases and preferably substantial pure Δ7 and Δ22 desaturases. b) incubating for a sufficient time to produce Δ7,22 bis dehydrocholesterol with high yield (more than 85%) c) extracting the sterols with a water inmiscible solvent d) recover pure Δ7,22 bis dehydrocholesterol.

It is possible to increase the efficiency of this transformation by increasing the solubility of cholesterol, using detergents or emulsifiers, i.e, tween 80, arabic gum, triton X100, etc.

For commercial production of cholesterol desaturases the genes of cholesterol desaturases may be transferred into another organism such as a bacterial species or yeast. The process would be similar to that now in use for making human insulin, bovine growth hormone or bovine chemosin.

In summary, the present invention describes the isolation of cholesterol desaturases, including Δ 7, Δ 22 and Δ 7, 22 desaturases, in cell free extracts from ciliate microorganisms as *Tetrahymena.*

It is believed that the applicants are the first ever to isolate this activity in cell free extracts and to use these fractions for treating cholesterol-containing foodstuffs.

The cell free extract can be used in the processing of animal food products, reducing cholesterol while simultaneously enhancing provitamin D availability.

The present invention utilizes the foregoing properties of the cell free extract from *Tetrahymena*, and other protozoa, to provide methods for treating cholesterol-containing foodstuffs (such as milk, egg and others) to achieve the following goals: (a) reduce the level of cholesterol in foodstuffs and (b) increase the level of cholesterol desaturated derivatives.

Selective introduction of a double bond on the seven position of a Δ5 sterol is important also for the synthesis of intermediates for important products, i.e, in the preparation of 1,25 dihydroxy vitamin D derivatives. A better possibility of synthesizing cholesterol desaturated derivatives from cholesterol is therefore of great importance.

In addition, due to the toxicity of the required chemicals, an environmentally friendly process for the synthesis of provitamin D and its analogs is desired.

In addition, simple and safe processes for producing unsaturated derivatives such as: Δ7 dehydrocholesterol (also named provitamin D3), Δ22 dehydrocholesterol and Δ7,22 bis dehydrocholesterol (a close analog of pro vitamin D2) having a high purity is also desired.

The desaturation of cholesterol at position 7 converts the same into provitamin D3 and pro vit D derivatives, which upon UV irradiation can be activated into vitamin D. This biotransformation has seldom been observed in nature.

Moreover, the biological system capable of performing cholesterol desaturations is not known: therefore, the attempts to fully explain the reaction mechanism have been frustrated.

The discovery of enzymes for converting cholesterol in vitamin D derivatives is desirable, because it is to be expected that it might be useful for:

1. The reduction of cholesterol in foodstuff with simultaneous increasing of more unsaturated derivatives.
2. The production of Δ7 dehydrosterol, Δ22 dehydrosterol and Δ7,22 dehydrosterol from sterol substrates.

The following examples merely illustrate the various embodiments now contemplated for practicing the invention but should not be construed to limit the invention.

## EXAMPLE 1

The use of a homogenate fraction from *Tetrahymena thermophila* for treatment of milk.

*Cells and cultures. Tetrahymena thermophila* wild type strain CU399 was maintained in axenic cultures. Inocula were prepared in medium containing 1% proteose peptone, 0.1% yeast extract (both from Difco Laboratories, Detroit, MI), 0.5% glucose and 0.01% Sequestrene (Ciba Geigy, Basel, Switzerland) and sterilized by autoclaving (121 °C, 20 min). Erlenmeyer flasks containing 100 ml of the same culture medium with 1mg of cholesterol added, were inoculated with 10 ml of a late log phase culture and incubated for 40 h, at 30 °C, on a rotary shaker at 150 rpm. Cell densities were estimated by counting appropriate dilutions of the cultures fixed with 2% formaldehyde in a Neubauer hemocytometer.

*Preparation of the homogenate fraction.* The ciliates were harvested by centrifugation at 15,000 g for 10 min, resuspended in 0.2 M $Na_2HPO_4$ buffer (pH 7.2), washed once in the same buffer and finally suspended in 10 mM Tris-HCl, pH 7.2, 250 mM sucrose, 5 mM $MgCl_2$, 1 mM EDTA (TES-Mg buffer). The suspensions were homogeneized by sonication (Vibracell, Sonics & Materials, Inc) in an ice-water bath until complete cell lysis (6 min, approximately). The suspension was centrifuged at 8,800 xg for 10 min, and the supernatant (homogenate) was tested for desaturase activity.

*Test for desaturase activity*. Cholesterol desaturase activity was assayed essentially as described for a fattyacid desaturase from *Tetrahymena termophila* (Bertram J. and Erwin J. J. Protozool. 28:127-131, 1981). 5 ml of the homogenate (approximately 250 mg of protein) was incubated with 0.01 mg unlabeled cholesterol as the carrier and 0.5 µCi of [$^3$H] cholesterol (46 Ci/mmol, Amersham Life Sciences, Buckinghamshire, U.K.). The mixture was incubated for 3 hours in a shaking water bath (150 rpm) at 30 ° C. Boiled homogenate preparations (10 min, 100 °C) were used as controls. The reaction was stopped by addition of one volume of 2 M sodium hydroxide prepared in methanol/water (1:1 v/v). The mixture was saponified during 1 hour at 60°C and further extracted.

*Analysis of sterols:* The unsaponifiable fraction which includes the sterols, was extracted twice with the same volume of chloroform, evaporated under nitrogen at 60°C, suspended in 100 µl of chloroform and separated by HPLC using a C18 column. Methanol/ water (95:5 v/v) was used as mobile phase at 1.3 ml/min. Sterols detection and quantification was monitored with an U.V. detector at 205 nm and 285 nm, or with a radiactivity detector as indicated above (Radiomatic Camberra Instruments).

To aid quantitation, stigmasterol (100 µg/ml) was added to the reaction mixture prior to saponification and used as internal standard. This sterol separates cleanly from cholesterol and its derivatives under the conditions employed.

***The extent of cholesterol desaturase activity was estimated from the conversion of the radiolabeled cholesterol added.***

*Bioconversion of radiolabeled cholesterol in milk.* Radiolabeled cholesterol ([$^3$H] cholesterol) was added from a 40 µCi/ml stock solution in ethanol, first by a 1:10 dilution in milk, followed by 2 hours incubation at 30 °C with shaking, to help partition of cholesterol into milk fat globules. Then, 10 ml of this cholesterol-milk intermediate suspension was added to 10 ml of the cell free homogenate and incubated 3 hours at 30 °C with gentle stirring. Total lipids from treated milk were saponofied 1 hour at 60°C. The unsaponifiable fraction was further extracted with hexane and separated by RP-HPLC monitored by radioactivity detector as indicated previously.

In Table 7, the change in cholesterol content before and after treatment of whole milk with and homogenate is displayed. In the conditions described in the present example, roughly 20 % of the cholesterol initially present is converted into the desaturated derivatives: Δ7- dehydrocholesterol (8%), Δ22- dehydrocholesterol (5%) and Δ7,22-didehydrocholesterol(3%). Thus, this data show that an homogenate of *Tetrahymena* cells can be used to reduce cholesterol concentration while at the same time increase the provision of desaturated cholesterol derivatives, including pro-vitamin D.

Table 7

| Cholesterol conversion in milk treated with a cell- free homogenate from *Tetrahymena termophila* | | | | |
|---|---|---|---|---|
| Incubation Time (Hours) | Cholesterol ug/ml (%) | Δ7 dehydrocholesterol ug/ml (%) | Δ22 dehydrocholesterol ug/ml (%) | Δ7,22 bisdehydrocholesterol ug/ml (%) |
| 0 | 71.5 (100) | ND | ND | ND |
| 3 | 55.7 (77.9) | 6.2 (8.7) | 4.1 (5.7) | 2.7 (3.8) |
| ND: no detected | | | | |

## Example 2

### The use of a cell free microsomal fraction from *Tetrahymena thermophila* for treatment of milk

***Preparation of a microsomal fraction from Tetrahymena termophila cells:* The procedure for *Tetrahymena* cell culture and homogenate preparation was identical to that set forth in example 1. The cell free homogenate was further centrifuged at 105,000 x g for 1.5 hours, and the resulting pellet was separated and suspended in the same buffer (TES-Mg buffer) to 5-10 mg/ml final protein concentration. All procedures were carried out at 4 ° C.**

*Assay for desaturase activity.* The procedure for assaying the cholesterol desaturase activity in microsomal fractions was essentially as described in example 1, except that 5 ml of the microsomal fraction was used. This volume was incubated with 0.01 mg unlabeled cholesterol as the carrier and 0.5 µCi of [$^3$H] cholesterol (46 Ci/mmol, Amersham Life Sciences, Buckinghamshire, U. K.). Sterols analysis, including saponification, extraction, detection and quantification were as described in example 1.

Figure 2 shows high performance liquid chromatograms showing the separation of radiolabeled cholesterol (peak 1) from its desaturated derivatives, Δ7-dehydrocholesterol (peak 2), Δ22- dehydrocholesterol (peak 3) and Δ7,22-didehydrocholesterol(peak4) before (control) and after (treated) incubation with a microsomal fraction obtained from *Tetrahymena* cells. As seen in Fig. 2, incubation of a microsomal fraction with $^3$H- cholesterol resulted in a decrease in the cholesterol level as well as an increase in the level of the desaturated cholesterol derivatives (Table 8).

A higher transformation of cholesterol present can be achieved using an inoculum of higher density or longer incubation periods.

*Treatment of milk with the microsomal fraction*. For this assay 10 ml of the microsomal fraction (protein concentration 5-10 mg/ml) was incubated with 10 ml of milk containing 0.5 μCi of [$^3$H] cholesterol (46 Ci/mmol, Amersham Life Sciences, Buckinghamshire, U.K). Pyridin cofactors (NAD, NADP, NADH and NADPH; 5mM each one) and ATP (5 mg/ml) werw added and the mixture was incubated at 30°C during 3 hours with gentle stirring (100 rpm). Sterols analysis, including saponification, extraction, detection and quantification were as described in example 1.

Utilizing this process there was a decrease in the amount of cholesterol and an increase in the unsaturated derivatives qualitatively and quantitatively similar to the changes reported in Example 1. In Table 8 the change in cholesterol content before and after treatment of whole milk with a cell free microsomal fraction is displayed. Roughly, 20 % of the cholesterol initially present in milk is converted into the desaturated derivatives: Δ7- dehydrocholesterol (5.2%), Δ22- dehydrocholesterol (6.5%) and Δ7,22-didehydrocholesterol(4.4%).

Table 8

| Cholesterol conversion in milk treated with a microsomal fraction from *Tetrahymena termophila* | | | | |
|---|---|---|---|---|
| Incubation Time (hours) | Cholesterol Ug/ml (%) | Δ-7 dehydro-cholesterol ug/ml (%) | Δ-22 dehydro-cholesteol ug/ml (%) | Δ-7-22 bisdehydro-cholesterol ug/ml (%) |
| 0 | 70 (100) | ND | ND | ND |
| 3 | 56.5 (80.7) | 5.2 (7.4) | 4.6 (6.5) | 3.1 (4.4) |
| **ND: no detected** | | | | |

## Example 3

### Use of a desaturase-enriched fraction from *Tetrahymena thermophila* for treatment of milk

*Preparation of a desaturase enriched fraction from Tetrahymena termophila cells:* **The procedure for *Tetrahymena* cell culture and microsomal preparation was identical to that set forth in Examples 1 and 2. 50 ml of the microsomal fraction (5 mg/ml, protein concentration) was mixed with a detergent solution (N-Octyltioglucoside,Sigma Chem, 5% w/v) to get a final detergent concentration ca 0.35% at pH 7.0. The detergent/ protein ratio was between 0.05 and 10 (w/w).**

The suspension was stirred during 4 hours at 15°C and centrifuged at 105,000 x g, 1.5 hours, to remove the non- solubilized components.

After ultracentrifugation and pellet separation solid $(NH_4)_2SO_4$ was added to the supernatant in a water bath set at 5°C until the concentration of $(NH_4)_2SO_4$ reached 45% w/v. The precipitate formed was centrifuged 10 min at 10,000 x g at 5°C. The supernatant was discarded and the precipitate was suspended in 2 ml of a phosphate buffer solution (0.1M, pH 7.2). This solution was dialyzed during 12 hours at 5°C with gentle stirring in 500 ml of buffer containing 0.1 M phosphate pH 7.2, 250 mM Sucrose, 0.1 mM EDTA.

After 3 changes of this solution, the sample was diluted to a final protein concentration of 10mg/ml. This procedure concentrated the cholesterol desaturase activity in a protein- enriched fraction which was tested for desaturase activity.

*Test for desaturase activity*. The procedure for assaying the cholesterol desaturase activity in desaturase-enriched fractions was essentially as described in example 1, except that 1.0 ml of the protein-enriched fraction (10.0 mg/ml protein) was used. This volume was incubated with 0.01 mg unlabeled cholesterol as the carrier and 0.5 μCi of [$^3$H] cholesterol (46 Ci/mmol, Amersham Life Sciences, Buckinghamshire, U.K.). Pyridin cofactors (NAD, NADP, NADH and NADPH; 5mM each one) and ATP (5 mg/ml) were added and the mixture was incubated at 30°C during 3 hours with gentle stirring (100 rpm). Sterols analysis, including saponification, extraction, detection and quantification were as described in example 1.

*Treatment of milk with the enriched fraction*. For the treatment of milk, 1.0 ml of the desaturase- enriched fraction was mixed with 1 ml of whole milk and incubated in the conditions previously described (3 hours at 30 °C, with gentle stirring (100rpm). Sterols analysis, including saponification, extraction, detection and quantification were as described in example 1.

Utilizing this process there was a decrease in the amount of cholesterol and an increase in the unsaturated derivatives similar to the changes reported in Examples 1 and 2. In Table 9 the change in cholesterol content before and after treatment of whole milk with a cell free desaturase-enriched fraction is displayed. Roughly, 30% of the cholesterol initially present is converted mostly into the desaturated derivatives: $\Delta 7$- dehydrocholesterol (11.3%), $\Delta 22$-dehydrocholesterol (9.1%) and $\Delta 7,22$-didehydrocholesterol(3.1 %).

Table 9

| Cholesterol conversion in milk treated with a desaturase-enriched fraction from *Tetrahymena termophila* | | | | |
|---|---|---|---|---|
| Incubation Time (hours) | Cholesterol ug/ml (%) | $\Delta 7$ dehydrocholesterol ug/ml (%) | $\Delta 22$ dehydrocholesteol ug/ml (%) | $\Delta 7,22$ bisdehydrocholesterol ug/ml (%) |
| 0 | 74.6 (100) | ND | ND | ND |
| 3 | 51.5 (69.0) | 8.3 (11.3) | 46.8 (9.1) | 5.2 (3.1) |
| **ND: no detected** | | | | |

## Example 4

### Use of a desaturase-enriched fraction from *Tetrahymena thermophila* for treatment of egg yolk

*Preparation of a desaturase enriched fraction from Tetrahymena termophila cells:* Proceed as in example 3. Test for desaturase activity: Proceed as in example 3

*Treatment of yolk with the desaturase enriched fraction.* For the treatment of yolk, 1.0 ml of the desaturase- enriched fraction was mixed with 10 ml of a 1/10 dilution of yolk and incubated in the conditions previously described for milk (3 hours at 30 °C, with gentle stirring (100rpm). Sterols analysis, including saponification, extraction, detection and quantification were as described in example 1.

Utilizing this process there was a decrease in the amount of cholesterol and an increase in the unsaturated derivatives similar to the changes reported in previous examples for milk. In Table 10 the change in cholesterol content before and after treatment of diluted yolk with a cell free desaturase-enriched fraction is displayed.

Table 10

| Cholesterol conversion in diluted yolk treated with a desaturase-enriched fraction from *Tetrahymena termophila* | | | | |
|---|---|---|---|---|
| Incubation time (hours) | Cholesterol mg/ml (%) | 7 dehydr$_{ochol.}$ mg/ml (%) | 22 dehydr$_{ochol.}$ mg/ml (%) | 7,22 bis dehydr$_{o\text{-}chol.}$mg/ml (%) |
| 0 | 1.40 (100) | ND | ND | ND |
| 3 | 0.91 (65.1) | 0.19 (14.0) | 0.11 (8.2) | 0.10 (7.7) |
| ND: no detected | | | | |

Example 5

Purification of $\Delta 7$ cholesterol dasaturase

*Step 1: Culturing a microorganism in a suitable medium, wherein said microorganism is capable of producing $\Delta 7$ cholesterol desaturases: Tetrahymena thermophila* wild type strain CU399 was maintained in axenic cultures. Inocula were prepared in medium containing 1% proteose peptone, 0.1% yeast extract (both from Difco Laboratories, Detroit, MI), 0.5% glucose and 0.01% Sequestrene (Ciba Geigy, Basel, Switzerland) and sterilized by autoclaving (121 °C, 20 min). Erlenmeyer flasks containing 100 ml of the same culture medium with 0,5 mg of 22 dehydrocholesterol added, were inoculated with 10 ml of a late log phase culture and incubated for 24 hs, at 30°C, on a rotary shaker at 150 rpm.

*Step 2: Desintegrating the culture and extracting same with a buffer solution:* The ciliates culture were harvested by centrifugation at 15,000 g for 10 min, resuspended in 0.2 M $Na_2HPO_4$ buffer (pH 7.2), washed once in the same buffer and finally suspended in 10 mM Tris-HCl, pH 7.2, 250 mM sucrose, 5 mM $MgCl_2$, 1 mM EDTA and 2uM of 2- mercaptoethanol (TES-Mg buffer). The suspensions were homogeneized by sonication (Vibracell, Sonics & Materials, Inc) in an ice-water bath until complete cell lysis (6 min, approximately). The suspension was centrifuged at 8,800 xg for 10 min, and the supernatant (homogenate) was further centrifuged at 105,000 x g for 1.5 hours, and the resulting pellet (microsomal fraction) was separated and suspended in the same buffer (TES-Mg buffer) to 5-10 mg/ml final protein concentration.

**This suspension was mixed with a detergent solution (N-Octyltioglucoside, Sigma Chem, 5% w/v) to get a final detergent concentration of 0.35% at pH 7.0. The detergent/protein ratio was between 0.05 and 10 (w/w).**

The suspension was stirred during 4 hours at 15°C and centrifuged at 105,000 x g, 1.5 hours, to remove the non- solubilized components. The supernatant is a solubilized protein solution

*Step 3: Recovering said cholesterol desaturase therefrom:* The solubilized fraction is concentrated to a small volume by 60% saturation with ammonium sulfate precipitation.

After centrifugation, the precipitated protein is dissolved in 50 mM sodium phosphate buffer (pH 7.4 and containing 10% of glycerol and 2uM of 2-mercaptoethanol) to give a protein concentration of 10 mg/ml.

The solution is then applied to a Phenyl sepharose column (Amersham Pharmacia Biotech) that has been equilibrated with the same buffer. The column is washed out with 50 mM sodium phosphate buffer (pH 7.4 and containing 10% of glycerol and 2uM of 2-mercaptoethanol) plus 1.0 M NaCl and then with the same buffer but without the addition of salt.

The eluted protein fraction was applied to a cation exchange chromatography (SP HiTrap column, Amersham Pharmacia Biotech) filled with 1.0 ml of SP Sepharose High performance (Pharmacia Biotech) equilibrated with 50 mM sodium phosphate buffer (pH 7.4).

After washing with 2 column volumes (2.0 ml) of the same buffer the enzyme was eluted by a linear NaCl gradient (0 to 1.0 M NaCl in 10 column volumes) in the same buffer (plus 10% glycerol)at a flow rate of 1.0 ml/min. 2 ml fraction was collected and desaturase activity was tested.

Fraction eluted between 200-400 mM of NaCl show maximal desaturase activity.

This fraction is applied to a Superose 12 FPLC column (HR 10-30) equilibrated with 50 mM sodium phosphate buffer (pH 7.4 and containing 10% of glycerol and 2uM of 2-mercaptoethanol). Activity fractions are eluted with the same buffer at a flow rate of 0.5 ml/min. the active fraction are collected and stored.

Example 6

Purification of Δ22 cholesterol desaturase

*Step 1: culturing a microorganism in a suitable medium, wherein said microorganism is capable of producing Δ22 cholesterol desaturases: Tetrahymena thermophila* wild type strain CU399 was maintained in axenic cultures. Inocula were prepared in medium containing 1% proteose peptone, 0.1% yeast extract (both from Difco Laboratories, Detroit, MI), 0.5% glucose and 0.01% Sequestrene (Ciba Geigy, Basel, Switzerland) and sterilized by autoclaving (121 °C, 20 min). Erlenmeyer flasks containing 100 ml of the same culture medium with 1.0 mg of cholesterol added, were inoculated with 10 ml of a late log phase culture and incubated for 24 hs, at 30°C, on a rotary shaker at 150 rpm.

*Step 2: Desintegrating the culture and extracting same with a buffer solution:* The ciliates were harvested by centrifugation at 15,000 g for 10 min, resuspended in 0.2 M $Na_2HPO_4$ buffer (pH 7.2), washed once in the same buffer and finally suspended in 10 mM Tris-HCl, pH 7.2, 250 mM sucrose, 5 mM $MgCl_2$, 1 mM EDTA (TES-Mg buffer). The suspensions were homogeneized by sonication (Vibracell, Sonics & Materials, Inc) in an ice-water bath until complete cell lysis (6 min, approximately). The suspension was centrifuged at 8,800 xg for 10 min, and the supernatant (homogenate) was further centrifuged at 105,000 x g for 1.5 hours, and the resulting pellet (microsomal fraction )was separated and suspended in the same buffer (TES-Mg buffer) to 5-10 mg/ml final protein concentration.

**This suspension was mixed with a detergent solution (N-Octyltioglucoside, Sigma Chem, 5% w/v) to get a final detergent concentration of 0.35% at pH 7.0. The detergent/ protein ratio was between 0.05 and 10 (w/w).**

The suspension was stirred during 4 hours at 15°C and centrifuged at 105,000 x g, 1.5 hours, to remove the non- solubilized components. The supernatant is a solubilized protein solution

*Step 3: Recovering said cholesterol desaturase therefrom.* The solubilized fraction obtained as in example 3 is concentrated to a small volume by 60% saturation with ammonium sulfate precipitation.

After centrifugation, the precipitated protein is dissolved in 50 mM sodium phosphate buffer (pH 7.4 and containing 10% of glycerol) to give a protein concentration of 10 mg/ml.

The solution thus obtained is applied to a column (XK16/20, Pharmacia Biotech) filled with 20ml of Q Source 30 (Pharmacia Biotech) equilibrated with buffer 50 mM sodium phosphate buffer (pH 7.4 and containing 10% of glycerol). After washing with 2 column volumes of the same buffer, a linear NaCl gradient (0 to 1.0 M, in 15 column volumes of the same buffer) was applied for the elution at a flow rate of 0.9 ml/min. 20 ml fractions were collected to test the $\Delta 22$ desaturase activity in 2 ml samples (with the addition of radiolabeled cholesterol)

Fraction between 5-15% of buffer B (50-150 mM of NaCl) showed maximal $\Delta 22$ desaturase activity.

The active fraction is collected, pooled and stored.

This active pool is submitted to gel filtration through a column of Superose 12 (HR 10/30, Pharmacia Biotech) and developed with buffer 50 mM sodium phosphate (pH 7.4 and containing 150 mM NaCl). Active fractions were eluted with the same buffer at a flow rate of 0.5 ml/min and collected for the assay of $\Delta 22$ desaturase activity.

Example 7

Production of substantially pure $\Delta 7$ dehydrocholesterol (provitamin D$_3$) from cholesterol

A substrate solution was prepared by dissolving pure cholesterol in hot ethanol to a concentration of 5 mM.

A substantial pure enzyme solution was prepared as was described in example 5. 0.9 ml of enzyme solution were mixed with 0.1 ml of substrate solution and incubated at 30°C.

After 3 hours of incubation in the same condition the reaction was stopped by addition of one volume of 2M sodium hydroxide prepared in methanol/water (1:1 v/v). The mixture was saponified during 1 hour at 60°C and further extracted with hexane.

The mixture of sterols ($\Delta 7$ dehydrocholesterol and cholesterol) present in the unsaponifiable fraction was separated into its components by silver nitrate column chromatography.

The column was eluted with a gradient of benzene-petroleum ether and the fraction was monitored by UV absorbance and HPLC analysis.

Pure fraction was collected, pooled and crystallized.

Example 8

Production of substantially pure $\Delta 7,22$ bis dehydrocholesterol from cholesterol

A substrate solution was prepared by dissolving pure cholesterol in hot ethanol to a concentration of 5 mM.

A substantial pure enzyme solution containing $\Delta 7$ desaturase was prepared as was described in example 5 (enzyme solution A)

A substantial pure enzyme solution containing $\Delta 22$ desaturase was prepared as was described in example 6 (enzyme solution B)

0.9 ml of enzyme solution A were mixed with 0.9 ml of enzyme solution B and 0.2 ml of substrate solution and incubated at 30°C.

After 1 hour of incubation in the same condition the reaction was stopped by addition of one volume of 2 M sodium hydroxide prepared in methanol/water (1:1 v/v). Sterols analysis, including saponification, extraction, detection and quantification were as described in example 7.

Example 9: Production of substantially pure 25 hydroxy $\Delta 7$ dehydrocholesterol from cholesterol

A substrate solution was prepared by dissolving pure 25 hydroxycholesterol in hot ethanol to a concentration of 5 mM.

A substantial pure enzyme solution containing $\Delta 7$ cholesterol desaturases was prepared as described in example 5.

0.9 ml of enzyme solution were mixed with 0.1 ml of substrate solution and incubated at 30°C.

After 1 hour of incubation in the same condition the reaction was stopped by addition of one volume of 2 M sodium hydroxide prepared in methanol/water (1:1 v/v). Sterols analysis, including saponification, extraction, detection and quantification were as described in example 7 showing a 75% of the conversion ratio from the starting material.

Example 10: Production of substantially pure 24 hydroxy $\Delta 7$ dehydrocholesterol from cholesterol

A substrate solution was prepared by dissolving pure 24(R) hydroxycholesterol in hot ethanol to a concentration of 5 mM.

A substantial pure enzyme solution containing Δ 7 desaturase was prepared as was described in example 5.

0.9 ml of enzyme solution were mixed with 0.1 ml of substrate solution and incubated at 30°C.

After 1 hour of incubation in the same condition the reaction was stopped by addition of one volume of 2 M sodium hydroxide prepared in methanol/water (1:1 v/v). Sterols analysis, including saponification, extraction, detection and quantification were as described in example 7 showing a 71% of the conversion ratio from the starting material.

Example 11. Production of substantially pure 1 alpha hydroxy Δ7 dehydrocholesterol from cholesterol

A substrate solution was prepared by dissolving pure 1-alpha hydroxycholesterol in hot ethanol to a concentration of 5 mM.

A substantial pure enzyme solution containing Δ 7 desaturase was prepared as was described in example 5.

0.9 ml of enzyme solution were mixed with 0.1 ml of substrate solution and incubated at 30°C.

After 1 hour of incubation in the same condition the reaction was stopped by addition of one volume of 2 M sodium hydroxide prepared in methanol/water (1:1 v/v). Sterols analysis, including saponification, extraction, detection and quantification were as described in example 7 showing a 68% of the conversion ratio from the starting material.

**Claims**

1. A cell free extract from Ciliate phylum microorganism, wherein said cell free extract contains sterol desaturase activities selected from the group comprising Δ7 and Δ22 sterol desaturases activities that catalyze desaturation of sterols.

2. A cell free extract of Claim 1, wherein said cell free extract is selected from the group consisting of cell free homogenate, microsomal fraction, desaturase-enriched fraction, substantial pure Δ7 cholesterol desaturases enzyme, substantial pure Δ22 cholesterol desaturases enzyme, or a combination thereof, all from Ciliata phylum microorganism.

3. A cell free extract of Claims 1 and 2, wherein the ciliate is selected from the group consisting of *Paremecium, Tetrahymena* and *Colpidium.*

4. A method for changing the sterols profiles of a cholesterol-containing foodstuff, wherein said method comprises at least the step of treating a foodstuff by incubating the same with a cell free extract of claim 1, wherein the cell free extract is selected from a group consisting of a homogenate fraction, microsomal fraction, desaturase-enriched fraction, substantial pure Δ7 cholesterol desaturase, substantial pure Δ22 cholesterol desaturase and combinations thereof, all obtained from a Ciliate phylum member in an effective amount to enable a change in the composition of the foodstuff, the change comprising: (a) reducing the level of cholesterol and (b) increasing the level of at least one cholesterol desaturated derivative.

5. The method of claim 4, wherein the level of cholesterol in the treated cholesterol-containing foodstuff is about 5 % to about 90% of the level of cholesterol in the cholesterol- containing foodstuff before the treating step.

6. The method of Claim 4, wherein the cholesterol- containing foodstuff is incubated with the cell free extract at a temperature of from 24°c to 37°c, at a pH of between 5.0 and 8.0, for a time period of from 1 to 24 hours.

7. The method of claim 4, wherein the cholesterol- containing foodstuff is selected from the group consisting of whole milk, egg and egg yolk.

8. The method of Claim 4, wherein the cholesterol desaturated derivative is selected from the group consisting of Δ7-dehydrocholesterol, Δ22-dehydrocholesterol and Δ7,22-bisdehydrocholesterol desaturated derivatives.

9. A process for manufacturing a dehydrosterol comprising:

(a) contacting a cell free extract of claim 1 with a sterol substrate, wherein the cell free extract is selected from a group consisting of a homogenate fraction, microsomal fraction, desaturase-enriched fraction, substantial pure Δ7 cholesterol desaturase enzyme, substantial pure Δ22 cholesterol desaturase enzyme and combinations thereof;

(b) incubating the cell free extract and de sterol substrate for a period of time enough to produce a dehydrosterol;

(c) recovering said dehydrosterol by solvent extraction and chromatographic purification.

10. The process of claim 9, wherein said dehydrosterol is selected from a group consisting of Δ7 dehydrocholesterol, Δ22 dehydrocholesterol and Δ7,22 bis dehydrocholesterol

11. A substantial pure Δ7 cholesterol desaturase enzyme from Ciliata phylum microorganism, wherein said enzyme is capable of catalyzing the conversion of a sterol substrate in Δ7 dehydrosterol by introducing a double bond at the position seven in the sterol molecules.

12. A substantial pure Δ7 cholesterol desaturase enzyme according to claim 11, the enzyme

(a) having a molecular weight of approximately 60 kDa by gel chromatography;
(b) having an optimum pH range for enzymatic activity between 6.5-8.5;
(c) having an optimum temperature range for enzymatic activity of 28°C to 35°C;
(d) being unaffected by metal ions such as $Ca^{+2}$, $Mn^{+2}$ and $Mg^{+2}$ ,EDTA concentrations and 2-mercaptoethanol;
(e) being inactivated after 1 minute at 100°C;
(f) being storage at -20°C by at least 6 months.

13. A substantial pure Δ7 cholesterol desaturase enzyme of Claim 11, wherein the ciliate is selected from the group consisting of *Paremecium, Tetrahymena* and *Colpidium.*

14. A process for preparing a substantial pure Δ7 cholesterol desaturase enzyme from Ciliata phylum microorganism according to claim 11, the process comprising the steps of:

(a) culturing a microorganism in a suitable medium, wherein said microorganism is capable of producing Δ7 cholesterol desaturases;
(b) disintegrating the culture and extracting the same with buffer solution containing, if necessary, non ionic surfactant or stabilizer as glycerol;
(c) subjecting the extract to a chromatography purification under suitable conditions; and
(d) eluting and recovering said Δ7 cholesterol desaturases enzymes.

15. A substantial pure Δ22 cholesterol desaturase enzyme from Ciliata phylum microorganism, wherein said enzyme is capable of catalyzing the conversion of a sterol substrate in Δ22 dehydrosterol by introducing a double bond at the position twenty-two in the sterol molecules.

16. A substantial pure Δ22 cholesterol desaturase enzyme according to claim 15, the enzyme

(a) having a molecular weight of approximately 60 kDa by gel chromatography;
(b) having an optimum pH range for enzymatic activity between 5.5-8.5;
(c) having an optimum temperature range for enzymatic activity of 28°C to 35°C;
(d) being unaffected by metal ions such as $Ca^{+2}$, $Mn^{+2}$ and $Mg^{+2}$ and EDTA concentrations;
(e) being inactivated after 1 minute at 100°C;
(f) being storage at -20°C by at least 6 months.

17. A substantial pure Δ22 cholesterol desaturase enzyme of Claim 15, wherein the ciliate is selected from the group consisting of *Paremecium, Tetrahymena* and *Colpidium.*

18. A process for preparing a substantial pure Δ22 cholesterol desaturase enzyme from Ciliata phylum microorganism according to claim 15, the process comprising the steps of:

(a) culturing a microorganism in a suitable medium, wherein said microorganism is capable of producing Δ22 cholesterol desaturases;
(b) disintegrating the culture and extracting the same with buffer solution containing, if necessary, non ionic surfactant or stabilizer as glycerol;
(c) subjecting the extract to a chromatography purification on a suitable chromatography conditions; and
(d) eluting and recovering said Δ22 cholesterol desaturases enzymes.

FIG. 1

Δ7,22-didehydrocholesterol

cholesterol

Δ7-dehydrocholesterol

Δ22-dehydrocholesterol

EP 1 251 167 A2

# FIG. 2

FIG. 3

Fig. 4

Fig. 5

EP 1 251 167 A2

Fig. 6

25